(19) Europäisches Patentamt
European Patent Office
Office européen des brevets

(11) Veröffentlichungsnummer : **0 498 180 B1**

(12) # EUROPÄISCHE PATENTSCHRIFT

(45) Veröffentlichungstag der Patentschrift :
**17.08.94 Patentblatt 94/33**

(21) Anmeldenummer : **92100635.9**

(22) Anmeldetag : **16.01.92**

(51) Int. Cl.$^5$ : **C07D 487/04,** C07D 493/04, G01N 33/52, // (C07D487/04, 237:00, 237:00), (C07D493/04, 307:00, 307:00)

(54) **3-Amino-benzol-1,2,4,5-tetracarbonsäuredihydrazide und ihre Verwendung als Chemilumineszenz-fähige Verbindungen sowie 3-Amino-benzol-1,2,4,5-tetracarbonsäuredianhydride.**

(30) Priorität : **05.02.91 DE 4103405**

(43) Veröffentlichungstag der Anmeldung :
**12.08.92 Patentblatt 92/33**

(45) Bekanntmachung des Hinweises auf die Patenterteilung :
**17.08.94 Patentblatt 94/33**

(84) Benannte Vertragsstaaten :
**AT BE CH DE FR GB IT LI SE**

(56) Entgegenhaltungen :
**CH-A- 419 166**
**DE-A- 2 924 066**
**PRAXIS DER NATURWISSENSCHAFTEN - CHEMIE Bd. 37, Nr. 1, 1988, Seiten 2-14**
**CHEMIE IN UNSERER ZEIT Bd. 24, Nr. 5, Oktober 1990, Weinheim, DE, Seiten 227 - 238**

(56) Entgegenhaltungen :
**CHEMICAL ABSTRACTS, Vol 59, No 3, 5 August 1963, Columbus, Ohio, US abstract no 2698D-2699A**
**CHEMICAL ABSTRACTS, Vol 55, No 21, 16 Oktober 1961, Columbus, Ohio,US, abstract no 21043E-H**
**CHEMICAL ABSTRACTS, Vol 55, no 14, 10 Juli 1961, Columbus, Ohio, US abstract no 13363B-E**

(73) Patentinhaber : **BASF Aktiengesellschaft**
**Carl-Bosch-Strasse 38**
**D-67063 Ludwigshafen (DE)**

(72) Erfinder : **Schefczik, Ernst, Dr.**
**Dubliner Strasse 7**
**W-6700 Ludwigshafen (DE)**
Erfinder : **Huemke, Klaus, Dr.**
**Buhlscher Hof 4**
**W-6701 Friedelsheim (DE)**

EP 0 498 180 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind 3-Amino-benzol-1,2,4,5-tetracarbonsäuredihyrazide und ihre Verwendung als Chemilumineszenz-fähige Verbindungen sowie 3-Amino-benzol-1,2,4,5-tetracarbonsäuredianhydride und Verfahren zur Herstellung der Dihydrazide und Dianhydride.

Aus der CH-A-419 166 sowie Chem. Abstr. 1961, 55, 13363b-e und 21043e-h sowie Chem. Abstr. 1963, 59, 2698d-2699a sind 1,4-substituierte Pyromellitsäureanhydride bekannt, die - soweit angegeben - zum Färben von Kunststoffen eingesetzt werden.

Unter Chemilumineszenz (CL) versteht man einen wohldefinierten Ausschnitt aus der Vielzahl bekannter Lumineszenzerscheinungen, bei dem die Emission von Licht im kausalen Zusammenhang mit einer bestimmten chemischen Reaktion steht.

Das Phänomen selbst ist heute weit über den engeren Bereich der Forschungsstätten, die sich mit dieser Thematik wissenschaftlich auseinandersetzen, hinaus bekannt geworden. Chemisches -"kaltes Licht" -ist bereits kommerziell in Form von Leuchtstäben erhältlich, die auf der chemischen Spaltung von Oxalestern basieren.

Über speziell konstruierte Luminometer hat die Chemilumineszenz bereits Einzug in analytische Laboratorien gehalten, und hier insbesondere in die medizinische Diagnostik. So lassen sich mit den heute verfügbaren elektronischen Möglichkeiten viele Substrate mit einer Genauigkeit erfassen, die bis vor wenigen Jahren noch als unerreichbar galt. ATP (Adenosintriphosphat) kann z.B. mit dieser Methodik bis in den Femto- und Attomolbereich nachgewiesen werden. Ähnliches gilt für Mg-Ionen, die im biochemschen Luciferin/Luciferase-System neben Sauerstoff und ATP zur Lichtemission erforderlich sind. Calcium läßt sich sogar in Mengen einwandfrei bestimmen, wie sie in einer einzelnen Körperzelle vorkommen.

Weitere durch CL erfaßbare Verbindungen sind z.B. fluoreszierende Kohlenwasserstoffe und Heterocyclen, Glucose im Blutserum und verschiedene Formen aktiven Sauerstoffs, wie er in organischen und anorganischen Peroxiden auftritt.

Die chemisch erzeugte Lumineszenz wird von den drei Kriterien chemische Reaktion, Energiekonversion und Lichtemission, die voneinander abhängig sind, bestimmt und zeigt damit, daß eine enge Verflechtung von physikalischen und chemischen Teilprozessen vorliegt.

Häufig wird die Lichtenergie im nicht-sichtbaren UV-Bereich emittiert. In diesem Fall ist es möglich, durch den Zusatz von echten Fluorophoren die Energie in sichtbares Licht umzuwandeln. Hier bieten sich besonders ausgedehnte aromatische Systeme mit konjugierten Elektronenpaaren an, z.B. 9,10-Diphenylanthracen oder 5,6,11,12-Tetraphenyltetracen. Aber auch das Porphyrinsystem des Blutes läßt sich auf diese Weise nutzen. Dazu wird meist das Haemin des Stierblutes eingesetzt.

Bei vielen lichterzeugenden Prozessen hat Sauerstoff eine wesentliche Bedeutung. Biolumineszenz wird durch Sauerstoffzufuhr erst ermöglicht. Ebenfalls von Lichtemission begleitet ist die Autoxidation von Kohlenwasserstoffen. Auch bei dem bekannten Leuchten des weißen Phosphors handelt es sich um CL, für die die Reaktion von PO mit Sauerstoff zum $PO_2$ verantwortlich gemacht wird. Die Reaktionen, die zu CL-Erscheinungen führen, sind fast alle oxidativer Natur.

Eine besonders gut untersuchte Stoffklasse stellen die Cyclodiacylperoxide dar. Hier findet man auch das Phthaloylperoxid, das mit einer Reihe von Fluorophoren brilliante CL ergibt.

Bekannter und wegen ihrer hohen Quantenausbeute besonders gut untersucht zeigt sich die CL der Dioxetane und deren Derivate.

Die unter starker CL verlaufende Perhydrolyse verschiedener Oxalsäurederivate stellt hinsichtlich ihrer Quantenausbeute (bis zu 0,34 Einstein/mol) und Einsetzbarkeit als chemische Lichtquelle gegenwärtig das günstigste System der bekannten CL-Systeme dar. Ihre CL läuft über die oxidative Bildung von Dioxetanen als Zwischenstufe und diverse Umlagerungsprodukte bis hin zur Bildung von angeregtem Kohlendioxid.

Als noch eine Stufe komplizierter erweist sich der Mechanismus der 3-Aminophthalsäurehydrazid-Chemilumineszenz. Das 3-Aminophthalsäurehydrazid bildet über die Diazachinon-Zwischenstufe und weitere Reaktionsschritte ein intramolekulares Dioxiran/Carbeniat-System, das hochfluoreszent ist und seine Energie bei einer intramolekularen Carbenoxidation als intensiv-blaue Chemi-Fluoreszenz abstrahlt, wobei es in das 3-Aminophthalsäuredianion übergeht.

Eine der am besten untersuchten Biolumineszenzen ist das Luciferin/Luciferase-System der amerikanischen Firefly, dem Leuchtkäfer ähnlich. Es hat zur ATP- bzw. Mg-Bestimmung Eingang in die medizinische Diagnostik gefunden.

Die Anwendung dieser Reaktion ist jedoch insofern mit Schwierigkeiten verbunden, als hierfür das Enzym Luciferase benötigt wird.

Der Schwerpunkt der neueren Entwicklungen liegt mehr auf dem analytischdiagnostischen Gebiet als auf dem Beleuchtungssektor.

Eine Übersicht des Standes der Technik enthält der Artikel von M. Steinfatt, "Chemilumineszenz: Grundlagen-Beispiele-Mechanismen", Praxis der Naturwissenschaften-Chemie 1/37. Jhg., 1988, S. 2-14, in dem auch auf die Notwendigkeit der Verbesserung vorhandener CL-Systeme hingewiesen wird.

Aufgabe der vorliegenden Erfindung ist es, leistungsfähige und möglichst gut zugängliche Verbindungen zur Verfügung zu stellen, die insbesondere bei der Anwendung in der Analytik ausgezeichnete Chemilumineszenz-Eigenschaften aufweisen.

Die Aufgabe wird gelöst durch 3-Amino-benzol-1,2,4,5-tetracarbonsäuredihyrazide der folgenden allgemeinen Formel I

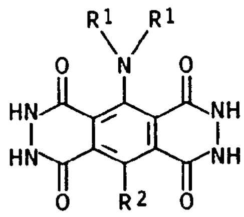

I

in der
$R^1$ = H, Methyl, Ethyl und
$R^2$ = H, $C_{1-6}$-Alkyl, $C_{1-6}$-Alkoxy

In der 6-Stellung erweist sich ein Elektronen-liefernder Substituent (+I und/oder +M-Effekt) als günstig.

Als besonders geeigneter Vertreter hat sich z.B. das 3-Amino-6-methylbenzol-1,2,4,5-tetracarbonsäuredihyrazid erwiesen.

Die nach der IUPAC-Nomenklatur als 3-Amino-benzol-1,2,4,5-tetracarbonsäuredihydrazide zu bezeichnenden Verbindungen können auch als 3-Aminopyromellitsäuredihydrazide bezeichnet werden.

Sie werden z.B. durch eine relativ einfache chemische Reaktion aus den 3-Amino-benzol-1,2,4,5-tetracarbonsäure-7:8;9:10-dianhydriden mit Hydrazinhydrat in z.B. ethanolischer Lösung hergestellt.

Die Reaktion läuft nach folgendem Schema ab:

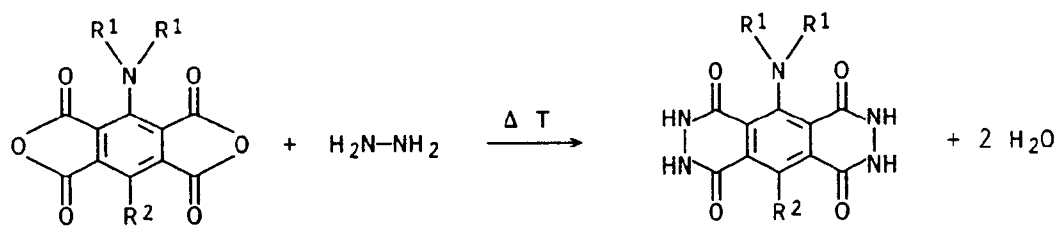


wobei $R^1$ und $R^2$ die oben angegebenen Bedeutungen haben.

Die Dianhydride der allgemeinen Formel II

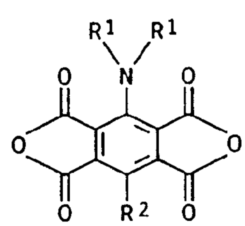

II

in der
$R^1$ = H, Methyl, Ethyl und
$R^2$ = H, $C_1$-$C_6$-Alkyl, $C_1$-$C_6$-Alkoxy
bedeuten,
sind Zwischenprodukte bei der Herstellung der erfindungsgemäßen Dihydrazide nach Formel I. Sie werden aus den substituierten Anilinen der allgemeinen Formel III

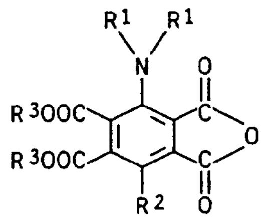

III

in der die Reste $R^1$ und $R^2$ die oben angegebene Bedeutung haben und $R^3$ gleich oder verschieden sind und $C_1$- bis $C_8$-Alkyl bedeuten, hergestellt, indem man die substituierten Aniline in Gegenwart von Säuren erhitzt, wobei direkt die Dianhydride erhalten werden. Die Dianhydride können aus den substituierten Anilinen aber auch durch stufenweise Reaktion, d.h. mit intermediärer Isolierung der Carbonsäuren, erhalten werden. Die Herstellung der substituierten Aniline ist in der DE 29 24 066 A1 beschrieben.

Es wurde nun gefunden, daß bei einer Oxidation der erfindungsgemäßen Dihydrazide mit Wasserstoffperoxid in alkalischer Lösung eine Chemilumineszenz auftritt.

Gegenstand der Erfindung ist daher auch die Verwendung der Stoffe gemäß obiger Formel I als Chemilumineszenz-fähige Verbindungen, insbesondere in der Analytik.

Beispiele

Herstellungsbeispiel

Darstellung des 3-Amino-6-methyl-benzo-1,2,4,5-tetracarbonsäure-7:8;9:10-dianhydrids

500 Teile 70 %ige Schwefelsäure werden bei 120°C gerührt. Dazu gibt man in Portionen 293 Teile 3-Methyl-4,5-bis-(methoxicarbonyl)-6-aminophthalsäureanhydrid. Man rührt 4 Stunden bei 125°C, läßt erkalten und fällt die Schmelze auf 500 Teile Eis. Nach dem Absaugen wird mit Eiswasser schwefelsäurefrei gewaschen und bei 120°C getrocknet.

Ausbeute: 210 Teile 3-Amino-6-methylpyromellitsäuredianhydrid in Form eines gelben Kristallpulvers. Die Verbindung läßt sich aus Essigsäure umkristallisieren und schmilzt nicht bis 350°C. Die Elementaranalyse ergab folgende Werte:

$C_{11}H_5NO_6$ (247) %

| | | | | |
|---|---|---|---|---|
| ber.: | C 53,5 | H 2,0 | N 5,7 | O 38,9 |
| gef.: | 53,3 | 2,2 | 5,7 | 38,8 |

Ähnliche Ergebnisse erhält man, wenn man die 70 %ige Schwefelsäure durch eine gleiche Menge 50 %ige, 60 %ige oder 80 %ige Schwefelsäure ersetzt.

1. Darstellung von 3-Amino-6-methyl-benzol-1,2,4,5-tetracarbonsäuredihyrazid

In einem 500 ml-Kolben mit Rührer, Rückflußkühler und Innenthermometer werden 20,0 g (0,081 mol) 3-Amino-6-methyl-benzol-1,2,4,5-tetracarbonsäure-7:8;9:10-dianyhdrid in 200 ml Ethanol unter Rückflußerhitzen gelöst. Die Lösung wird heiß filtriert und anschließend gekühlt. Bei Raumtemperatur werden 50,0 g (0,74 mol) Hydrazinhydrat (ca. 100 %ig) innerhalb von 10 Minuten zugetropft. Ein zunächst auftretender gelber Niederschlag geht bei weiterer Zugabe wieder in Lösung. Die Reaktion ist leicht exotherm. Nach dem überschreiten der Exothermiespitze wird die Mischung auf Rückflußtemperatur erhitzt und 2 bis 3 Stunden auf dieser Temperatur gehalten. Es entsteht ein feinverteilter rotbrauner Niederschlag. Nach Stehen über Nacht wird der Niederschlag abgesaugt und intensiv mit kaltem Ethanol gewaschen.

Wegen der Toxizität des überschüssigen Hydrazinhydrats (carcinogen) sind entsprechende Sicherheitsvorschriften zu beachten.

Man trocknet bei 50°C im Vakuum und kristallisiert aus wäßrigem Ethanol um. Die Substanz kristallisiert als Monohydrat.

Ausbeute: 26,0 g (92,5 % der Theorie)

Schmp.: > 320°C

| Elementaranalyse: | | C | H | N | O |
|---|---|---|---|---|---|
| [%] | theor.: | 45,1 | 4,0 | 27,6 | 23,3 |
| | gef.: | 44,7 | 4,4 | 28,0 | 22,9 |

IR (KBr): 3334 (m); 1711 (s); 1625 (m); 1415 (m); 1242 (m); 1106 (w); 705 (w) cm exp-1.
H-NMR (DMSO): 2,31 (s); 2,51 (s); 2,74 (s); 2,93 (s), 4,46 (d); 6,05 ... 7,85 (m).

2. Erzeugung von Chemilumineszenz durch Peroxidoxidation in alkalischer Lösung

Benötigte Reagenzien oder Maßlösungen:

a) 1/10000 molare Lösung des 3-Amino-6-methyl-benzol-1,2,4,5-tetracarbonsäure-7:8;9:10-dihydrazids in VE-Wasser
b) 1/100 molare wäßrige Kalilauge
c) 1 %ige wäßrige Wasserstoffperoxidlösung
d) 1/10000 molare wäßrige Haemin-Lösung (aus Stierblut)
e) 1/1000 molare alkoholische Lösung von 9,10-Diphenylanthracen
f) 1/10000 molare wäßrige Lösung von 3-Aminophthalsäurehydrazid (Luminol)

Versuch 1:

10 ml a) werden mit 3 ml b) versetzt. Nach Zugabe von 2 ml d) werden 2 ml c) zugesetzt. In einem leicht abgedunkelten Raum läßt sich ein intensives blau-violettes Licht erkennen. Die Intensität nimmt mit der Zeit ab. Das Leuchten ist aber noch 10 Minuten nach dem Beginn der Reaktion zu erkennen.

Versuch 2:

10 ml a) werden mit 3 ml b) versetzt. Nach Zugabe von 2 ml e) werden 2 ml c) zugesetzt. In einem leicht abgedunkelten Raum läßt sich ein intensives blaues Leuchten erkennen, dessen Intensität mit der Zeit abnimmt. 10 Minuten nach Beginn der Reaktion ist immer noch ein blaßblauer Schimmer zu erkennen.

Vergleich:

10 ml f) werden mit 3 ml b) versetzt. Nach Zugabe von 2 ml d) werden 2 ml c) zugesetzt. In einem stark abgedunkelten Raum läßt sich ein schwaches blaues Leuchten erkennen, das rasch an Intensität verliert und nach etwa 5 Minuten nicht mehr zu erkennen ist.

Aus diesen Versuchen wird deutlich, daß die erfindungsgemäßen Substanzen deutlich bessere CL-Eigenschaften aufweist als das bekannte 3-Aminophthalsäurehydrazid.

Der erfindungsgemäße Stoff kann in an sich bekannter Weise in oder an einen Eiweißkörper über die Aminogruppe in 3-Position ($R^1$ = H) eingebaut werden. Anschließende Oxidation mit Peroxiden in alkalischer Lösung führt zu Lichtemissionen, die in der biochemischen bzw. medizinischen Analytik brauchbar sind, insbesondere zur Bestimmung des ATP und von Ca- oder Mg-Ionen. Eine Darstellung derartiger Anwendungen in der klinischen Chemie, Biochemie und Medizin findet sich in "Chemie in unserer Zeit", 24.Jhg., 1990, Nr. 5, S. 227-238, worauf Bezug genommen wird.

## Patentansprüche

1. 3-Amino-benzol-1,2,4,5-tetracarbonsäuredihydrazide der folgenden allgemeinen Formel I

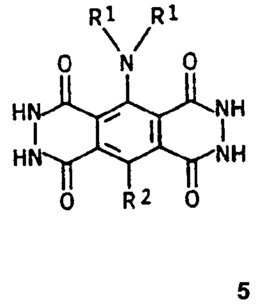

I

in der
$R^1$ = H, Methyl, Ethyl und
$R^2$ = H, $C_{1-6}$-Alkyl, $C_{1-6}$-Alkoxy
bedeuten.

2. Stoff der Formel I nach Anspruch 1, wobei $R^1$ = H und $R^2$ = $C_{1-3}$-Alky ist.

3. Stoff der Formel I nach Anspruch 1, der 3-Amino-6-methyl-benzol-1,2,4,5-tetracarbonsäuredihydrazid ist.

4. Verfahren zur Herstellung von 3-Amino-benzol-1,2,4,5-tetracarbonsäuredihydraziden der allgemeinen Formel I nach Anspruch 1,
dadurch gekennzeichnet, daß man 3-Amino-benzol-1,2,4,5-tetracarbonsäure-7:8;9:10-dianhydride der allgemeinen Formel II

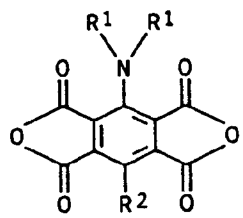 II

in der
$R^1$ = H, Methyl, Ethyl und
$R^2$ = H, $C_{1-6}$-Alkyl, $C_{1-6}$-Alkoxy
bedeuten,
mit Hydrazinhydrat in Lösung umsetzt, wobei die Reaktionslösung gegebenenfalls erwärmt wird.

5. 3-Amino-benzol-1,2,4,5-tetracarbonsäure-7:8;9:10-dianhydride der allgemeinen Formel II gemäß Anspruch 4.

6. Verfahren zur Herstellung von 3-Amino-benzol-1,2,4,5-tetracarbonsäure-7:8;9:10-dianhydriden der allgeneinen Formel II gemäß Anspruch 5,
dadurch gekennzeichnet, daß man substituierte Aniline der allgemeinen Formel III

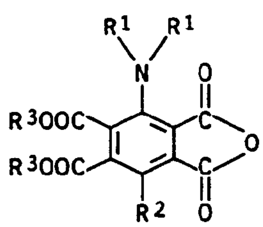 III

in der die Reste $R^1$ und $R^2$ die oben angegebene Bedeutung haben und $R^3$ gleich oder verschieden sind und $C_1$- bis $C_8$-Alkyl bedeuten, in Gegenwart von Säuren erhitzt.

7. Verwendung der Stoffe gemäß den Ansprüchen 1 bis 3 als Chemilumineszenz-fähige Verbindungen, insbesondere in der Analytik.

8. Verwendung nach Anspruch 7, dadurch gekennzeichnet, daß die Chemilumineszenz-fähigen Verbindungen in der biochemischen und medizinischen Analytik angewendet werden.

## Claims

1. A 3-aminobenzene-1,2,4,5-tetracarboxylic dihydrazide of the following formula I

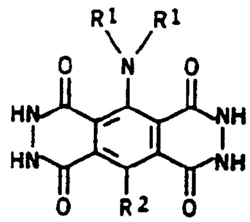

I

where $R^1$ is H, methyl or ethyl and $R^2$ is H, $C_{1-6}$-alkyl or $C_{1-6}$-alkoxy.

2. A substance of the formula I as claimed in claim 1, where $R^1$ is H and $R^2$ is $C_{1-3}$-alkyl.

3. A substance of the formula I as claimed in claim 1, which is 3-amino-6-methylbenzene-1,2,4,5-tetracarboxylic dihydrazide.

4. A process for preparing 3-aminobenzene-1,2,4,5-tetracarboxylic dihydrazides of the formula I as claimed in claim 1, which comprises reacting 3-aminobenzene-1,2,4,5-tetracarboxylic dianhydrides of the formula II

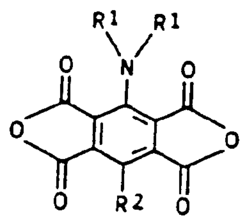

II

where $R^1$ is H, methyl or ethyl and $R^2$ is H, $C_{1-6}$-alkyl or $C_{1-6}$-alkoxy, with hydrazine hydrate in solution, heating the reaction solution if necessary.

5. A 3-aminobenzene-1,2,4,5-tetracarboxylic dianhydride of the formula II as claimed in claim 4.

6. A process for preparing 3-aminobenzene-1,2,4,5-tetracarboxylic dianhydrides of the formula II as claimed in claim 5, which comprises heating substituted anilines of the formula III

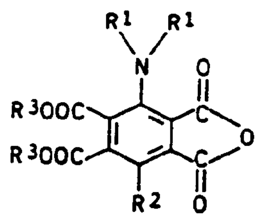

III

where $R^1$ and $R^2$ have the abovementioned meanings, and $R^3$ is identical or different $C_1$-$C_8$-alkyl, in the presence of acids.

7. The use of the substances as claimed in any of claims 1 to 3 as compounds capable of chemiluminescence especially in analysis.

8. The use as claimed in claim 7, wherein the compounds capable of chemiluminescence are used in biochemical and medical analysis.

## Revendications

1. Dihydrazides de l'acide 3-amino-benzène-1,2,4,5-tétracarboxylique de la formule générale I suivante :

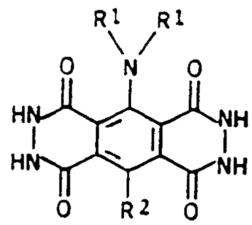

I

dans laquelle
$R^1$ représente un atome d'hydrogène, le radical méthyle, éthyle et
$R^2$ représente un atome d'hydrogène, un radical alkyle en $C_1$-$C_6$, alcoxy en $C_1$-$C_6$.

2. Substance de la formule I suivant la revendication 1, dans laquelle $R^1$ représente un atome d'hydrogène et $R^2$ représente un radical alkyle en $C_1$-$C_3$.

3. Substance de la formule I suivant la revendication 1, caractérisée en ce qu'elle est le dihydrazide de l'acide 3-amino-6-méthyl-benzène-1,2,4,5-tétracarboxylique.

4. Procédé de préparation de dihydrazides de l'acide 3-amino-benzène-1,2,4,5-tétracarboxylique de la formule générale I suivant la revendication 1, caractérisé en ce que l'on fait réagir des 7:8,9:10-dianhydrides de l'acide 3-amino-benzène-1,2,4,5-tétracarboxylique de la formule générale II

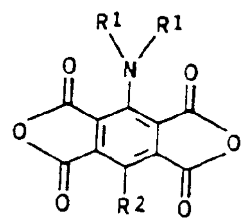

II

dans laquelle
$R^1$ représente un atome d'hydrogène, le radical méthyle, éthyle et
$R^2$ représente un atome d'hydrogène, un radical alkyle en $C_1$-$C_6$, alcoxy en $C_1$-$C_6$,
avec de l'hydrate d'hydrazine, en solution, où l'on chauffe éventuellement la solution réactionnelle.

5. 7:8,9:10-Dianhydrides de l'acide 3-amino-benzène-1,2,4,5-tétracarboxylique de la formule générale II suivant la revendication 4.

6. Procédé de préparation de 7:8,9:10-dianhydrides de l'acide 3-amino-benzène-1,2,4,5-tétracarboxylique de la formule générale II suivant la revendication 5, caractérisé en ce que l'on chauffe des anilines substituées de la formule générale III

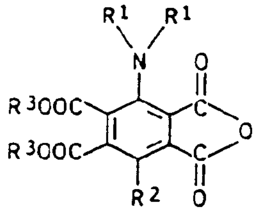

III

dans laquelle les symboles $R^1$ et $R^2$ ont les significations qui leur ont été attribuées ci-dessus et les symboles $R^3$, sont identiques ou différents et représentent des radicaux alkyle en $C_1$ à $C_8$, en présence d'acides.

7. Utilisation des substances suivant l'une quelconque des revendications 1 à 3 à titre de composés capables de chimiluminescence, plus particulièrement dans le domaine de l'analytique.

**8.** Utilisation suivant la revendication 7, caractérisée en ce que les composés capables de chimiluminescence sont exploités dans le domaine de l'analytique biochimique et médicale.